# EUROPEAN PATENT SPECIFICATION

(11) **EP 3 056 559 B1**
(45) Date of publication and mention of the grant of the patent: **10.07.2019**
(21) Application number: 16161790.7
(22) Date of filing: 16.05.2012
(51) Int. Cl.: C11D 1/88, C07K 1/14

(54) **COMPOSITIONS FOR SOLUBILIZING CELLS AND/OR TISSUE**
ZUSAMMENSETZUNGEN ZUR SOLUBILISIERUNG VON ZELLEN UND/ODER GEWEBE
COMPOSITIONS POUR SOLUBILISER DES CELLULES ET/OU UN TISSU

(43) Date of publication of application: 17.08.2016
(62) Divisional of application: 12876815.7
(73) Proprietor: THE REGENTS OF THE UNIVERSITY OF CALIFORNIA, Oakland, CA 94607-5200 (US); DX Biosciences, Inc., San Diego, CA 92101 (US)
(72) Inventor: Mitragotri, Samir, Santa Barbara, CA 93111 (US); Hwang, Byeong Hee, Incheon (KP); Lebovitz, Russell M., San Francisco, CA 94105 (US)
(74) Representative: Lawrie, Donald James

(56) References cited:
- WO-A2-2010/093861
- US-A- 4 214 908
- US-A- 5 639 630
- US-A1- 2004 191 854
- US-A1- 2009 269 380
- US-A1- 2009 304 799
- US-A1- 2010 226 983
- US-A1- 2010 261 176
- US-A1- 2012 064 136
- US-B2- 7 608 278
- MANDEEP SINGH BAKSHI ET AL: "Mixed micelle behavior of poly(ethylene glycol) alkyl ethers with series of monomeric cationic, phosphonium cationic, and zwitterionic surfactant", COLLOID AND POLYMER SCIENCE, vol. 285, no. 1, 2006, pages 101-106, XP019442834,
- S. PALIWAL ET AL: "One-step acquisition of functional biomolecules from tissues", PROCEEDINGS OF THE NATIONAL ACADEMY OF SCIENCES, vol. 107, no. 33, 2010, pages 14627-14632, XP055124735,

## Description

This invention was made with government support under federal grant number W81XWH-06-01-00400 awarded by the United States Army. The United States Government has certain rights in this invention.

### BACKGROUND

Skin is the "window" to the body. Skin is unique among the body's organs for several reasons: (1) skin is the largest organ of the human body; (2) skin is directly exposed to the environment; (3) skin is an excellent excretory organ; (4) skin is the most visible and accessible organ of the body; and (5) skin is a highly active immune organ of the body.

Skin has another important quality: The molecular profile of skin has information that is valuable for physiological monitoring of, among other things, small organic molecules, proteins, DNA, RNA, and lipids. Much can be learned from skin's molecular profiling. For example, pathogens (e.g., bacteria) that grow on skin may allow for forensic identification. Skin's molecular profile may reveal environmental factors to which the body has been passively exposed. These environmental factors may range from the mundane, e.g., allergens, toxins, and cosmetic products, to the industrial and/or agricultural, e.g., industrial solvents, fertilizers, and pesticides, to the dangerous, e.g., explosives and other warfare agents.

Skin's molecular profile may also reveal factors to which the body has been actively exposed. More particularly, skin's molecular profile may reveal what the body has consumed. For example, abused substances (e.g., illegal drugs or narcotics) and therapeutic drugs (e.g., tramadol, fluconazole, barbitals, and anabolic steroids) may be found in skin weeks after consumption.

Skin's molecular profile may also aid diagnosis of conditions and diseases. For example, skin cholesterol is a proxy of the extent of arterial blocks. Glycation of skin collagen is an indicator of a history of diabetes. Skin deposition of β-amyloids may indicate the existence and extent of Alzheimer's disease. And skin globular proteins (e.g., IgE) may indicate allergies to specific allergens.

Several methods exist for sampling biomolecules from skin. For example, one current method is skin biopsy. However, skin biopsy is invasive and analysis is difficult. Practically speaking, skin biopsy is designed for well-equipped experts and, thus, its use in a point-of-care setting is limited. Another current method for sampling biomolecules from skin, tape stripping, suffers from these same limitations and is generally unacceptable because of variability in results. Yet another current method for sampling biomolecules from skin is taking a skin swab. While desirable because of its simplicity, a skin swab is superficial in its depth of inspection, and qualitative in its results. Finally, tissue has been subjected to ultrasound in the presence of surfactants such as sorbitans ("SPANs"), polyoxyethelene sorbitans combined with fatty acids ("tweens"), cetyl trimethylammonium bromide ("CTAB"), and their mixtures. See U.S. Patent No. 6,589,173 issued to Mitragotri et al. However, SPANs, tweens, and CTAB, individually and collectively, have been found to be unsuitable to recover skin constituents. Sorbitans and tweens, which are nonionic surfactants, are mild and non-denaturing in character, but are ineffective to solubilize skin tissue. CTAB, a cationic surfactant, is effective to solubilize skin tissue, but unsuitably denatures proteins, profoundly changing properties of biomolecules in solution, rendering them unusable for functional purposes. WO 2010/093861 discloses a system, method and sevice for tissue-based diagnosis.

Along with providing a cornucopia of information, skin can also be a host to myriad undesirable cosmetic conditions, such as age spots, skin tags, seborrheic keratosis, scar tissues, xanthomas, non-cancerous hyperproliferative conditions, surface bumps, and scaly patches; and therapeutic conditions such as skin tumors, actinic keratosis, leukoplakia, and surface cancers relating to Barrett's esophagus and right-colon pre-cancer plaque. For these conditions, solubilization and remodeling or removal may be the primary concern, with or without subsequent diagnostic processing.

A need exists for compositions for skin sampling, as well as for mucosal membrane and other tissue sampling, which, when used in conjunction with applied energy, at least partially solubilize such skin, mucosal membrane, and other tissue. A further need exists to preserve the functionality and structural integrity of analytes, including biomolecules, obtained from the solubilized skin, mucosal membrane, and other tissue.

### SUMMARY

In a first embodiment, a composition is provided, the composition comprising:
a zwitterionic surfactant, comprising: 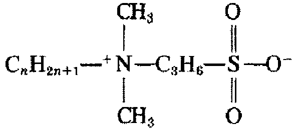 wherein n = 14 ; and
a non-ionic surfactant, comprising:

   H(CH₂)ₐO(CH₂CH₂O)_{b}H

   wherein a = 16, and wherein b = 2.

Accordingly, in a reference composition n = 12 (corresponding to N-dodecyl-N,N-dimethyl.3-ammonio-1-propanesulfonate or "DDPS"), a = 16, and b = 10 (corresponding to polyoxyethylene (10) cetyl ether or "Brij C10").

Accordingly, in a reference composition n = 14 (corresponding to N-tetradecyl-N,N-dimethyl-3-ammonio-1-propanesulfonate or "TPS"), a = 12, and b = 4 (corresponding to polyoxyethylene (4) lauryl ether or "Brij 30"). In an embodiment, n = 14 (TPS), a = 16, and b = 2 (corresponding to polyoxyethylene (2) cetyl ether or "Brij 52"). In another embodiment, n = 14 (TPS), a = 16, and b = 10 (Brij C10).

Accordingly, in a reference composition n = 16 (corresponding to N-hexadecyl-N,N-dimethyl-3-ammonio-1-propanesulfonate or "HPS"), a = 12, and b = 4 (Brij 30). In another embodiment, n = 16 (HPS), a = 16, and b = 2 (Brij 52). In another embodiment, n = 16 (HPS), a = 16, and b = 10 (Brij C10).

Accordingly, in a reference composition n = 18 (corresponding to N-octadecyl-N,N-dimethyl-3-ammonio-1-propanesulfonate or "OPS"), a = 12, and b = 4 (Brij 30). In another embodiment, n = 18 (OPS), a = 16, and b = 2 (Brij 52). In another embodiment, n = 18 (OPS), a = 16, and b = 10 (Brij C10). In another embodiment, n = 18 (OPS), a = 16, and b = 20 (corresponding to polyoxyethylene (20) cetyl ether or "Brij 58").

In a second embodiment, a non-surgical method is provided for solubilizing cosmetically remodeling and/or removing tissue on a patient's skin, comprising applying energy to a region of interest on the skin; and contacting the region with a tissue solubilizing composition of the first embodiment, wherein b can also be 10.

In a third embodiment, a tissue solubilizing composition for use in a surgical or diagnostic method practised on the human or animal body for recovering analytes from mucosal membrane, skin, or other tissue is provided, the method comprising: applying energy to a region of interest on the mucosal membrane, skin, or other tissue containing at least one analyte; contacting the region with a tissue solubilizing composition according to the first embodiment, wherein b can also be 10, thereby solubilizing at least some of the mucosal membrane, skin, or other tissue containing at least one analyte; and collecting the at least one analyte from the solubilized mucosal membrane, skin, and other tissue.

In a fourth embodiment a tissue solubilizing composition for use in a surgical or therapeutic method for solubilizing, remodelling , and/or removing tissue on or beneath a patient's skin is provide:
wherein the tissue solubilizing composition is according to embodiment 1, where b can also be 10; and
wherein the surgical or therapeutic method comprises:
   applying energy to a region of interest on the skin; and
   contacting the region with the tissue solubilizing composition.

### BRIEF DESCRIPTION OF THE DRAWINGS

In the accompanying figures, experimental data are given that, together with the detailed description provided below, describe example embodiments of the claimed invention.
**Figure 1** illustrates in graphical form the total protein (mg/ml) recovered from porcine skin when the porcine skin is contacted with various combinations of zwitterionic and nonionic surfactants in the presence of ultrasound.
**Figure 2** illustrates in graphical form the soluble protein (mg/ml) recovered from porcine skin when the porcine skin is contacted with various combinations of zwitterionic and nonionic surfactants in the presence of ultrasound.
**Figure 3** illustrates the dependence on concentration of the protein extraction efficacy from porcine skin of TPS:Brij C10.
**Figure 4** illustrates in graphical form the preserved activity of the intracellular enzyme glyceraldehyde 3-phosphate dehydrogenase (GAPDH) in various combinations of zwitterionic and nonionic surfactants.
**Figure 5** illustrates in graphical form the combined dependence of soluble protein extraction efficacy and GAPDH stability in various combinations of zwitterionic and nonionic surfactants.
**Figure 6** illustrates the total protein recovery from Human Epidermal Keratinocyte (HEK) cells at t = 0 (solid bars), t = 4 hours at 4 °C (diagonal bars), and t = 4 hours at RT (cross-hatched bars), using various reagents.
**Figure 7** illustrates the GAPDH activity measured in HEK cells solubilized in various reagents, at t = 0 (solid bars), t = 4 hours at 4 °C (diagonal bars), and t = 4 hours at RT (cross-hatched bars).
**Figure 8** illustrates the GAPDH activity measured in HEK cells solubilized in various reagents, at room temperature at t = 4 hours (solid bars), t = 24 hours (diagonal bars), 72 hours (cross-hatched bars), and 7 days (horizontal bars).
**Figure 9** illustrates the total protein recovered from homogenized mouse skin using various reagents.
**Figure 10** illustrates the specific GAPDH activity measured in homogenized mouse skin using various reagents.

### DETAILED DESCRIPTION

In one embodiment, a composition is provided, the composition comprising:
a zwitterionic surfactant, comprising: 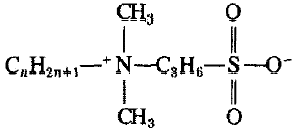 wherein n = 14; and
a non-ionic surfactant, comprising:

   H(CH₂)ₐO(CH₂CH₂O)_{b}H

   wherein a = 16, and wherein b = 2 or 10.

Accordingly, in a reference composition the zwitterionic surfactant comprises DPS and the nonionic surfactant comprises at least one of Brij 30, Brij 35, Brij 52, Brij C10, and Brij 58.

Accordingly, in a reference composition the zwitterionic surfactant comprises DDPS and the nonionic surfactant comprises at least one of Brij 30, Brij 35, Brij 52, Brij C10, and Brij 58.

In another embodiment, the zwitterionic surfactant comprises TPS and the nonionic surfactant comprises at least one of Brij 52 or Brij C10.

Accordingly, in a reference composition the zwitterionic surfactant comprises HPS and the nonionic surfactant comprises at least one of Brij 30, Brij 35, Brij 52, Brij C10, and Brij 58. In one embodiment, the combination of HPS:Brij 35 is excluded.

Accordingly, in a reference composition the zwitterionic surfactant comprises OPS and the nonionic surfactant comprises at least one of Brij 30, Brij 35, Brij 52, Brij C10, and Brij 58.

The zwitterionic surfactant and the nonionic surfactant may be dissolved in a buffer solution. The buffer solution may comprise, for example, one or more of phosphate-buffered saline (PBS) (pH = 7.2-7.6), tris-buffered saline (pH = 7.4 - 8.0), tris-hydrochloride (7.0 - 9.0), and ethylenediaminetetraacetic acid (EDTA) (pH = 7.4 - 9.0). Thus, the surfactant combination may have a pH of greater than about 7.0 in buffer solution, between about 7.0 and 9.0 in buffer solution, between about 7.4 and 9.0 in buffer solution, between about 7.4 and 8.0 in buffer solution, and between about 7.2 and 7.6 in buffer solution.

The zwitterionic surfactant and the nonionic surfactant may be present in a total concentration of between about 0.01% and about 10% (w/v) in the buffer solution. For example, the zwitterionic surfactant and the nonionic surfactant may be present in a total concentration of about 0.01% to about 5% (w/v) in the buffer solution, including total concentrations of about 0.1% (w/v) to about 2% (w/v) in the buffer solution, about 1% (w/v) in the buffer solution, and about 0.1% (w/v) to about 0.5% (w/v) in the buffer solution. In one embodiment, the zwitterionic surfactant and the nonionic surfactant are present in a total concentration of about 0.5% (w/v) in the buffer solution. In another embodiment, the zwitterionic surfactant and the nonionic surfactant may be present in a ratio of about 3:1 to about 1:3 or about 3:2 to about 2:3. In one embodiment, the zwitterionic surfactant and the nonionic surfactant may be present in a ratio of about 1:1.

The zwitterionic surfactant:nonionic surfactant composition may have several applications.

For example, in one embodiment, a method is provided for solubilizing and remodeling and/or removing tissue on or beneath a patient's skin, comprising applying energy to a region of interest on the skin; and contacting the region with a tissue solubilizing composition comprising at least one of: TPS and at least one of Brij Brij 52 or Brij C10.

In another embodiment, a method for recovering analytes from mucosal membrane, skin, or other tissue is provided, the method comprising: applying energy to a region of interest on the mucosal membrane, skin, or other tissue containing at least one analyte; contacting the region with a tissue solubilizing composition, the tissue solubilizing composition comprising at least one of: TPS and at least one of Brij 52 or Brij C10, thereby solubilizing at least some of the mucosal membrane, skin, or other tissue containing at least one analyte; and collecting the at least one analyte from the solubilized mucosal membrane, skin, and other tissue.

In some embodiments, "other tissue" may include breast, prostate, eye, vagina, bladder, nail, hair, colon, testicles, intestine, lung, brain, pancreas, liver, heart, bone, or aorta wall.

In some embodiments, an "analyte" may include any biomolecule, drug, small molecule, warfare agent, environmental contaminant, microbe, and the like that is present in or on the tissue and can be extracted from the tissue of interest.

In some embodiments, "biomolecules" may include proteins (e.g., disease biomarkers such as cancer biomarkers, antibodies: IgE, IgG, IgA, IgD, or IgM, and the like), peptides, lipids (e.g., cholesterol, ceramides, and fatty acids), nucleic acids (e.g., RNA and DNA), small molecules (e.g., glucose, urea, and creatine), small molecule drugs or metabolites of small molecule drugs, microbes, inorganic molecules, elements, and ions (e.g., iron, Ca²⁺, K⁺, Na⁺, and the like). In some embodiments, the biomolecule is exclusive of glucose and cancer markers.

In some embodiments, "drugs" may include abused drugs, such as, for example, cocaine, heroin, methyl amphetamine, and prescription drugs taken in excess of dosage, or taken without a prescription (e.g., painkillers such as opioids); and therapeutic drugs, such as, for example, tramadol, fluconazole, barbitals, and anabolic steroids.

In some embodiments, "warfare agents" may include any molecule, compound, or composition of either biological or chemical origin that may be used as a weapon. Non-limiting examples of warfare agents include explosives, nerve gases (e.g., VX and Sarin), phosgene, toxins, spores (e.g., anthrax), and the like.

In some embodiments, "environmental contaminants" may include any molecule, compound, or composition that can be detrimental to an individual, e.g., when at concentrations elevated above a risk threshold. Examples include water pollutants (e.g., fertilizers, pesticides, fungicides, insecticides, herbicides, heavy metals, and halides), soil pollutants (e.g., fertilizers, pesticides, fungicides, insecticides, herbicides, heavy metals, and halides), and air pollutants (e.g., NOₓ, SOₓ, greenhouse gases, persistent organic pollutants, particulate matter, and smog).

In some embodiments, solubilizing the target cells and tissue includes the application of energy. In some embodiments, the energy may be applied by any number of suitable methods, including mechanical (e.g., abrasion, shear, vacuum, pressure, suction, ultrasound), optical (e.g., laser), thermal, and electrical energy. However, in one embodiment, the energy does not include externally supplied thermal energy (i.e., heat). Suitable energy applicators are disclosed in U.S. Patent Application Serial Nos. 12/664,994, 13/126,105, and 13/095,771, each of which is incorporated by reference herein in its entirety.

The compositions may be used for solubilizing cells for *in vitro* protein recovery. In addition to effective dissolution of cells, the compositions may provide a benefit of preservation of bioactivity. The compositions may also possess the ability to quickly solubilize various tissues, including those with durable mechanical properties, such as skin. To aid in the preservation of bioactivity, a protease inhibitor may be included in the compositions. However, with or without the addition of protease inhibitors, the compositions may be able to preserve the biological activity of proteins. The compositions are also applicable *in vivo.* In particular, the compositions may be able to recover labile phosphoproteins with RPPA, thus opening the possibility of quickly and non-invasively probing multiple signaling pathways.

According to this disclosure, the compositions may be useful as antibacterial compositions. Thus, a method for inhibiting the growth and reproduction of bacteria and/or treating a bacterial infection is provided, the method comprising applying an antibacterial composition to an area that is subject to attack by the bacteria, the antibacterial composition comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58. The method may further include the application of energy.

According to the present disclosure, a method is provided for solubilizing cells and/or tissues, the method comprising contacting the cells and/or tissues with a composition, the composition comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58. Accordingly, proteins, including cytosolic proteins, nuclear proteins, and surface proteins may be recovered from the solubilized cells and/or tissues. In some embodiments, such as, for example, embodiments where it is desirable to preserve biological activity of the proteins, the composition may further optionally comprise a protease inhibitor.

According to the present disclosure, the compositions may be used to probe protein functional states and related skin cell signaling pathways. Skin cell signaling pathways may be stress-induced, and may change over minutes to hours. Phosphorylation is a highly labile post-translational modification that regulates many aspects of protein function. The ability to probe these functional states in the epidermis necessitates a fast and efficient method to solubilize and isolate phosphoproteins. Accordingly, a method is provided for recovering signaling proteins from skin cells, the method comprising: contacting the skin cells with a composition comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, to provide solubilized signaling proteins; and subjecting the solubilized signaling proteins to reverse phase protein array.

Accordingly, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful for, among other things, disaggregating, solubilizing, and stabilizing cell components to be used as disease biomarkers, forensic biomarkers, or both. In one embodiment, the compositions are useful to disaggregate, solubilize, and stabilize components from living tissues *in situ,* from freshly resected tissues, frozen resected tissues, preserved paraffin embedded tissues, tissue and cell extracts and cultured cells derived from cell lines or resected tissues, and from exogenous agents such as viruses, bacteria, and prions.

Accordingly the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful to solubilize, remodel, and remove diseased tissue on or beneath the surface of the skin, or elsewhere within the body. For example, the compositions may be useful to solubilize, remodel, and remove tissue hosting precancerous conditions such as actinic keratosis, leukoplakia, Barretts esophagus, and right-colon pre-cancer plaque, and surface cancers arising from any of these precancerous conditions. Other therapeutic uses may include solubilizing and removing tumors from a variety of surface or deep sites, or treating tumor surgical margins to remove any residual tumor cells at these sites. In some instances, after treating tumors with the compositions and solubilizing the constituent tumor markers, the immune system may detect the dissolved tumor markers and initiate a potent anti-tumor immune response against these markers, leading to regression of the local tumor, as well as destruction of any systemic tumor cells carrying the detected tumor markers.

Accordingly, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful for treating skin lesions and damaged skin with therapeutic molecules and drugs that are unable to penetrate an intact outer skin barrier. More particularly, the compositions described herein may be useful to enhance absorption of topical therapeutics by removing diseased tissue, inflammatory cells, and thickened, hyper-keratinized skin that may block access to otherwise effective topical therapies. An example of such a use is as a pretreatment for psoriasis topical therapies, since psoriasis lesions typically have a hardened top layer or hyperkeratosis that inhibits absorption. Accordingly, such treatment may include perturbation of the outer skin barrier using the compositions, and in some circumstances applied energy, to disrupt the barrier by disaggregation and solubilization of barrier cells and tissues, followed by application of the therapeutic molecules and drugs directly to the surface of the barrier-perturbed skin. Example therapeutic molecules and drugs may include, for example, DNA-based drugs, RNA-based drugs, protein-based drugs, peptide-based drugs, lipid-based drugs, carbohydrate-based drugs, small molecule drugs, nanoparticle based drugs, liposome-encapsulated drugs, and combinations of such classes of drugs.

Accordingly, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful for introducing therapeutic or diagnostic molecules and drugs into the body and bloodstream by disrupting the outer skin layer. In one embodiment, the introducing may include perturbation of the outer skin barrier using the compositions, and in some circumstances applied energy, to disrupt the barrier by disaggregation and solubilization of barrier cells and tissues, followed by application of the therapeutic or diagnostic molecules directly to the surface of the barrier-perturbed skin.

Accordingly, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful to remove malignant and benign growths and obstructions in other organs in the body or within the central and peripheral nervous systems, including the eye, middle ear, brain, spinal cord, nerve roots, and ganglia. Since the compositions may dissociate and dissolve diseased tissue directly after injection through a thin needle or catheter, the compositions may allow ablative surgery in areas that are not accessible to either open surgery or even to minimally invasive surgical instruments (such as in the vascular system, including arteries and coronary arteries).

Furthermore, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful to diminish or reduce intra-abdominal and peritoneal adhesions by dissolving specific bands of adherent tissue between intra-abdominal tissues and organs.

Moreover, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful for wound debridement. Accordingly, the treatment may include contacting the compositions, and in some circumstances applied energy, to a wound's surrounding tissue to remove unhealthy tissue, including, for example, necrotic eschar and fibrinous slough.

Furthermore, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful as a bio-glue to enhance post-operation healing.

Moreover, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful to promote oral and dental hygiene. For example, the compositions may be useful to soften and/or dissolve hard and soft deposits on teeth and dentures.

Furthermore, the compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful to solubilize, remodel, and remove cosmetically relevant structures on or beneath the surface of the skin. For example, the compositions may be useful for treating aged, scarred, and UV-damaged skin, and removing and/or remodeling age spots, skin tags, seborrheic keratosis, scar tissues, xanthomas, non-cancerous hyperproliferative conditions, surface bumps, and scaly patches. In one embodiment, the use may include perturbation of the outer skin barrier using the compositions, and in some circumstances applied energy, to disrupt the barrier by disaggregation and solubilization of barrier cells and tissues. The compositions may be introduced to deeper layers of skin to facilitate disaggregation, solubilization, and removal of structures associated with wrinkling, scarring, or both, of the skin surface. Other cosmetic uses include dermal peel or skin bleaching. The compositions may be used to remove discoloration of the skin associated with previous injury, UV-damage, or aging. The perturbation may be followed by application of therapeutic drugs or natural products and other cosmetic compositions that are believed or known to increase the smoothness, elasticity, and resilience of skin. Such cosmetic compositions may include, for example, elastin or its peptides (e.g., V-V-P-Q), collagen or its peptides, resveratrol, idebenone, co-enzyme Q10, acetyl hexapeptide-3, glycosaminoglycans, palmitoyl pentapeptide-4, sodium hyaluronate, and the like, and combinations thereof.

Cell components recovered using the compositions may be introduced into biochemical assays to detect, quantify, and identify specific biomarkers associated with specific diseases. The biochemical assays may include all molecular diagnostic assays for detecting DNA, RNA, proteins, peptides, lipids, carbohydrates, and small molecules, both endogenous and exogenous. The biochemical assays to be used include PCR, ELISA, chromatography, gel analysis, electrophoresis, Western Blots, Southern Blots, Northern Blots, and other methods used in clinical laboratories for identification of molecular biomarkers of disease.

The compositions comprising at least one of: (1) DPS and at least one of Brij 30, Brij 35, Brij 52, Brij C10, and Brij 58; (2) DDPS and at least one of Brij 30, Brij 35, Brij 52, Brij C10, and Brij 58; (3) TPS and at least one of Brij 30, Brij 35, Brij 52, Brij C10, and Brij 58; (4) HPS and at least one of Brij 30, Brij 35, Brij 52, Brij C10, and Brij 58; and (5) OPS and at least one of Brij 30, Brij 35, Brij 52, Brij C10, and Brij 58, may be useful in the treatment of mucosal and skin lesions. Such use may involve impregnating an abrasive and absorbent swab (similar to a for-daily-use facial exfoliating sponge) with one or more of the compositions, and applying the swab to the region of interest with medium pressure in a twisting or back-and-forth motion onto target tissue for a predetermined time. The depth may be controlled by the amount of pressure, as well as application time. After the lesion is dissolved and the released biomarkers are absorbed into the pad, the swab and the dissolved tissue may be collected and sent to, e.g., a pathology lab, for biomarker and other analyte analysis.

### EXAMPLES

Certain embodiments are described below in the form of examples. It is impossible to depict every potential application of the invention. Thus, while the embodiments are described in considerable detail, it is not the intention to restrict or in any way limit the scope of the appended claims to such detail, or to any particular embodiment.

### Example 1: Materials

DPS, DDPS, TPS, HPS, and OPS were procured from Sigma-Aldrich as zwitterionic surfactants. Brij 35, Brij 52, Brij C10, and Brij 58 were procured from Sigma-Aldrich as nonionic surfactants. Brij 30 was procured from TCI America. All solutions were prepared by adding 0.25% w/v zwitterionic surfactant and 0.25% w/v nonionic surfactant (0.25% v/v B30), for a total concentration of 0.5% w/v in PBS (pH = 7.4). A positive control was prepared by placing 1% v/v Triton X-100 (TX-1) in PBS.

### Example 2: Protein recovery from porcine skin

Protein was recovered from porcine skin as a model tissue. Skin was procured in frozen form from Lampire Biological Laboratories Inc., and stored at -70 °C. Two hours before use, skin was thawed at room temperature (RT) and cut into small pieces (2.5 cm × 2.5 cm). Skin pieces were stripped off from subcutaneous fat and used without visible scratches or abrasions. Protein recovery was carried out by mounting the skin piece on a Franz diffusion cell (FDC) assembly (tissue exposure area of 1.77 cm²; Permegear). The receiver chamber of the FDC was filled with PBS and the donor chamber was filled with 1 mL of surfactant combination as a sampling buffer. This buffer also acted as the coupling fluid between the ultrasound transducer and the tissue. Protein recovery was performed at RT with a 600-W probe sonicator (Sonics & Materials) operating at a frequency of 20 kHz. The ultrasound transducer was placed at a distance of 5 mm from the tissue surface and an ultrasonic intensity of 2.4 W/cm² at 50% duty cycle was applied for 3 min. The sampling buffer, now containing solubilized tissue constituents, was aspirated and kept at -70 °C until analysis.

The solubilization ability for each surfactant formulation disclosed herein was quantified by the concentrations of total protein and solubilized protein (mg/ml). Supernatants were isolated from the samples using a centrifuge operating at 10,000 × g and 4 °C for 15 min. The solubilized protein amount was measured in the sample supernatant by using a colorimetric detection kit (Micro BCA Protein Assay Kit; Pierce).

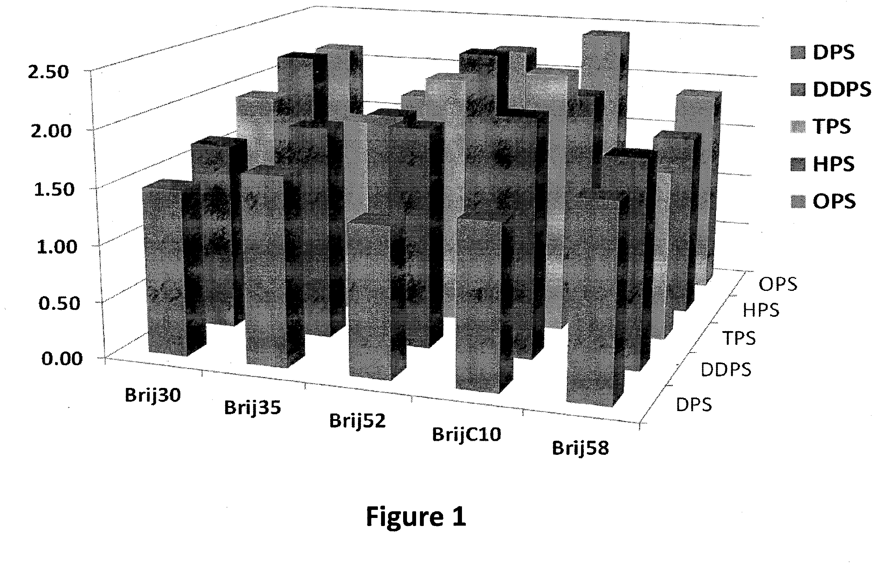

**Table 1** illustrates the total protein (mg/ml) recovered from porcine skin when the porcine skin is contacted with various combinations of zwitterionic and nonionic surfactants in the presence of ultrasound. **Figure 1** illustrates the same results in graphical form.

**Table 1**

| | Brij 30 | Brij 35 | Brij 52 | Brij C10 | Brij 58 |
|---|---|---|---|---|---|
| DPS | 1.47 ± 0.26 | 1.67 ± 0.20 | 1.33 ± 0.46 | 1.44 ± 0.23 | 1.69 ± 0.33 |
| DDPS | 1.68 ± 0.25 | 1.90 ± 0.43 | 1.95 ± 0.44 | 2.11 ± 0.29 | 1.84 ± 0.23 |
| TPS | 1.95 ± 0.26 | 1.79 ± 0.05 | 2.23 ± 0.25 | 2.33 ± 0.36 | 1.52 ± 0.19 |
| HPS | 2.19 ± 0.05 | 1.68 ± 0.26 | 2.32 ± 0.29 | 1.98 ± 0.31 | 1.64 ± 0.39 |
| OPS | 2.15 ± 0.54 | 1.71 ± 0.19 | 2.22 ± 0.23 | 2.42 ± 0.36 | 1.88 ± 0.32 |

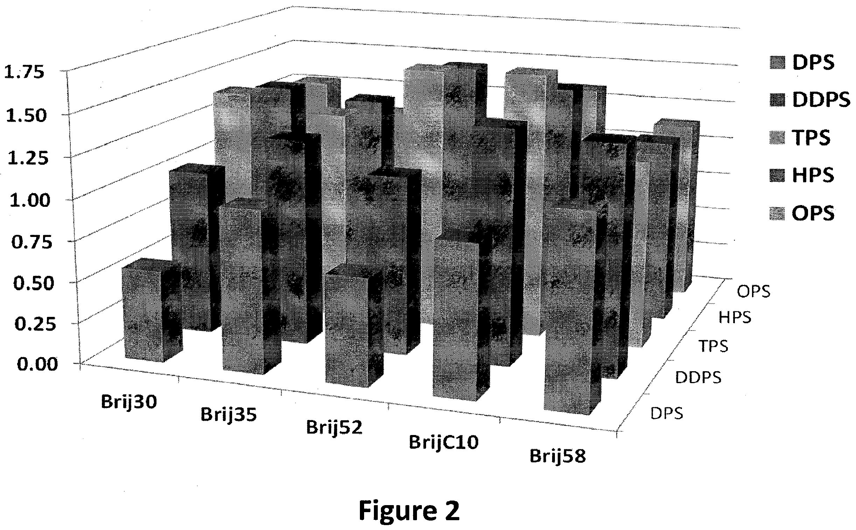

**Table 2** illustrates the soluble protein (mg/ml) recovered from porcine skin when the porcine skin is contacted with those same combinations of zwitterionic and nonionic surfactants in the presence of ultrasound. **Figure 2** illustrates the results in graphical form.

**Table 2**

| | Brij 30 | Brij 35 | Brij 52 | Brij C10 | Brij 58 |
|---|---|---|---|---|---|
| DPS | 0.56 ± 0.04 | 0.99 ± 0.30 | 0.65 ± 0.03 | 0.91 ± 0.13 | 1.15 ± 0.07 |
| DDPS | 1.02 ± 0.12 | 1.27 ± 0.19 | 1.10 ± 0.12 | 1.44 ± 0.16 | 1.39 ± 0.22 |
| TPS | 1.40 ± 0.26 | 1.31 ± 0.15 | 1.63 ± 0.16 | 1.64 ± 0.33 | 1.16 ± 0.25 |
| HPS | 1.34 ± 0.27 | 1.29 ± 0.26 | 1.55 ± 0.13 | 1.45 ± 0.18 | 1.15 ± 0.29 |
| OPS | 1.27 ± 0.28 | 1.09 ± 0.14 | 1.06 ± 0.25 | 1.34 ± 0.20 | 1.14 ± 0.16 |

### Example 3: Concentration dependence

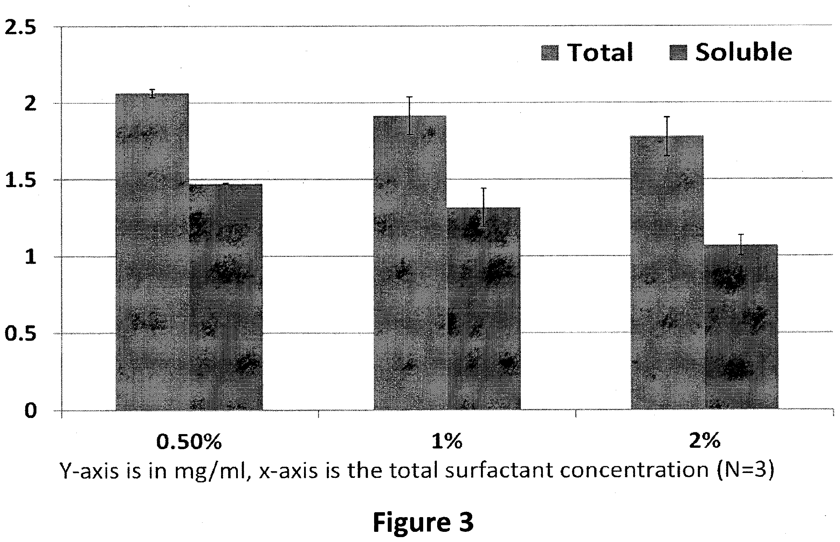

TPS:Brij C10 was investigated for concentration effect. The surfactant concentration was tested at total concentrations of 0.5%, 1%, and 2% of TPS:Brij C10, at a 1:1 ratio. **Figure 3** illustrates the dependence on concentration of TPS:Brij C10 on the soluble and total protein extraction efficacy from porcine skin.

### Example 4: Enzyme protection

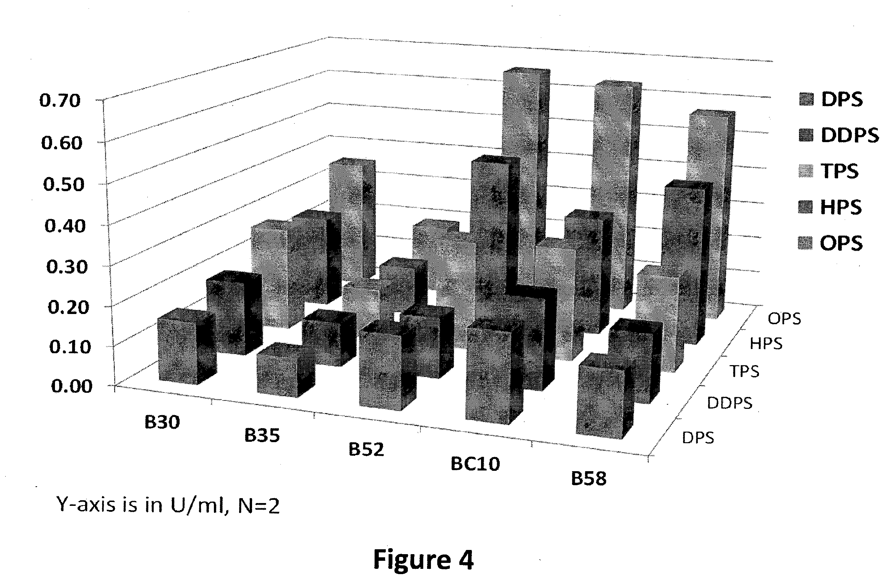

GAPDH from rabbit was procured as a model enzyme from Sigma-Aldrich. GAPDH powder was dissolved in ddH₂O (Milipore) to 90 U/ml and distributed into an E-tube, then stored at -80 °C until use. Enzyme protection was carried out by incubation of GAPDH in each combination disclosed herein, compared to that in PBS as a positive control. The concentration of GAPDH was fixed to 0.5 U/ml. GAPDH was incubated at 37 °C for 10 min. The activity of the GAPDH enzyme (U/ml) was assessed by KDalert™ GAPDH Assay Kit (Ambion, Inc, TX, USA). **Figure 4** illustrates in graphical form the preserved activity of GAPDH in each of the various combinations of zwitterionic and nonionic surfactants.

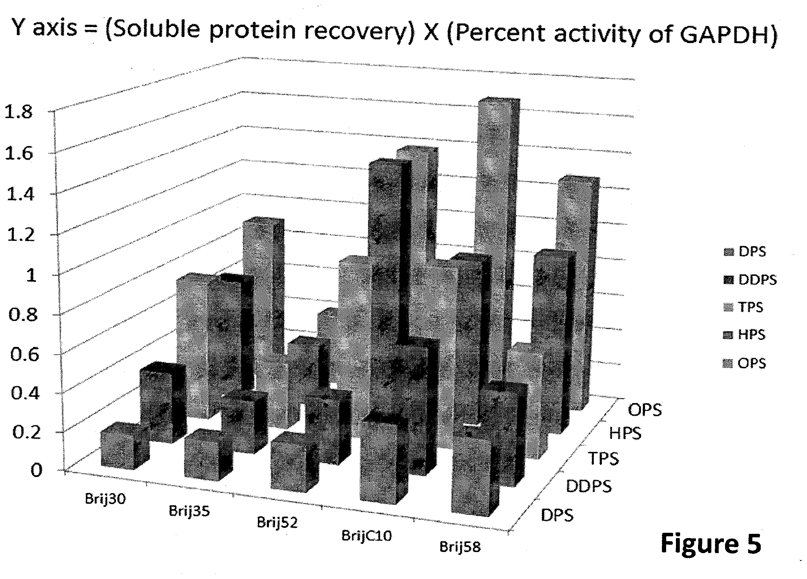

GAPDH activities were multiplied by soluble protein recovery. **Figure 5** illustrates in graphical form the combined dependence of soluble protein extraction efficacy and GAPDH stability in each of the various combinations of zwitterionic and nonionic surfactants.

### Example 5: Cell Solubilization

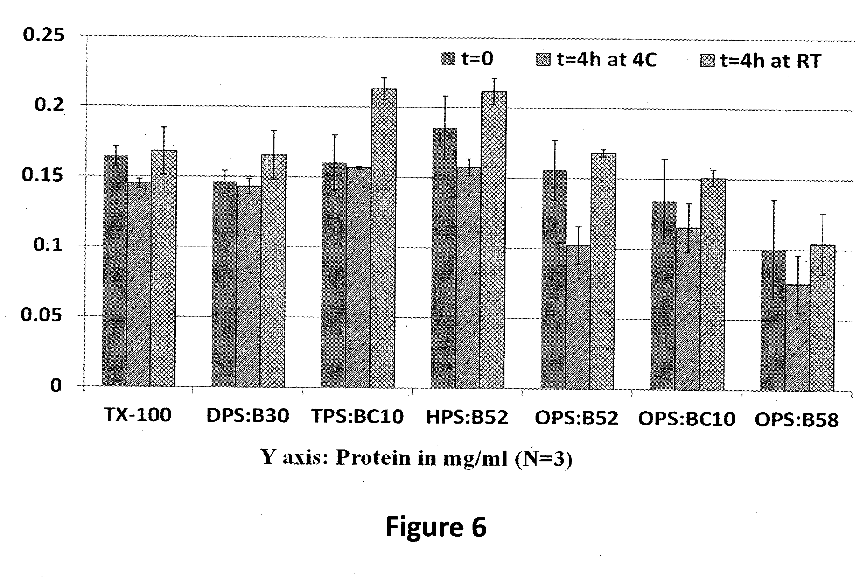

HEK cells (HEKa-APF, Invitrogen, CA, USA) were cultured in a Corning® cell culture treated flask with a Vent Cap (75 cm² Rectangular Canted Neck, Corning). The HEK cells were grown between passage 3 to 8 in the EpiLife® Medium with 60 µM calcium added by Human Keratinocyte Growth Supplement. **Figure 6** illustrates the total protein recovery from HEK cells at t = 0 (solid bars), t = 4 hours at 4 °C (diagonal bars), and t = 4 hours at RT (cross-hatched bars), using DPS:Brij 30, TPS:Brij C10, HPS:Brij 52, OPS:Brij 52, OPS:Brij C10, and OPS:Brij 58, as compared to TX-1.

### Example 6: In vitro assay

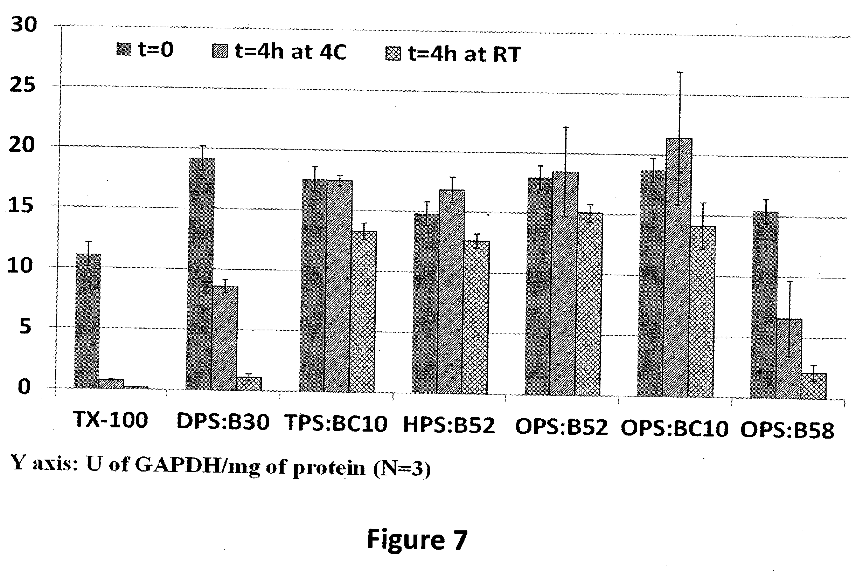

Total protein in solubilized cells was analyzed using a Micro BCA Protein Assay Kit (Fisher Scientific, PA, USA). Trypsinized cells were counted by hemocytometer (Fisher Scientific, PA, USA) and added to 96-well plates. After overnight growth, the medium was removed and replaced by 100 µl of lysis agents (TX-I, DPS:Brij 30, TPS:Brij C10, HPS:Brij 52, OPS:Brij 52, OPS:Brij C10, and OPS:Brij 58). **Figure 7** illustrates the GAPDH activity measured in HEK cells solubilized in those same reagents, at t = 0 (solid bars), t = 4 hours at 4 °C (diagonal bars), and t = 4 hours at RT (cross-hatched bars).

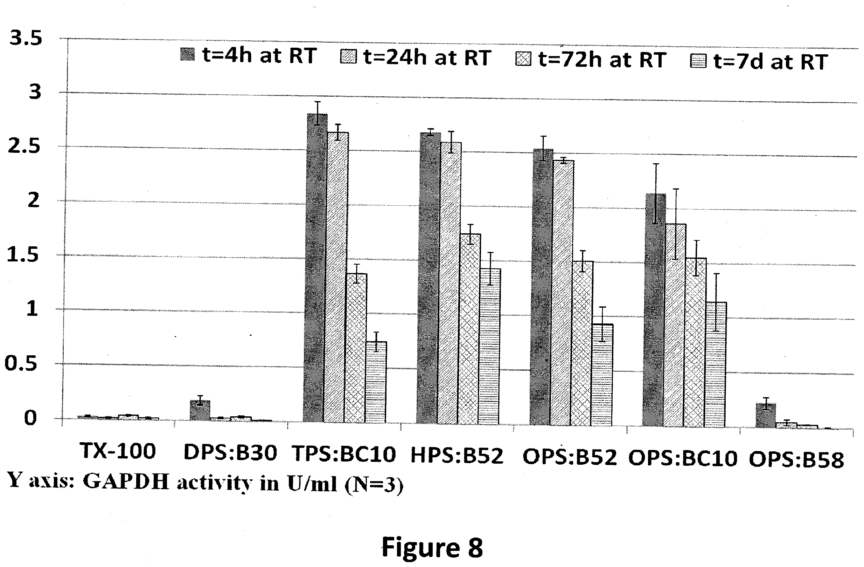

For further study of stability, the enzymes were incubated for one day at RT, three days at RT, and seven days at RT. **Figure 8** illustrates the GAPDH activity measured at RT, at t = 4 hours (solid bars), t = 24 hours (diagonal bars), t = 72 hours (cross-hatched bars), and 7 days (horizontal bars).

### Example 7: Tissue Solubilization

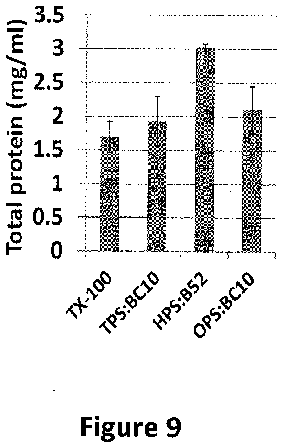

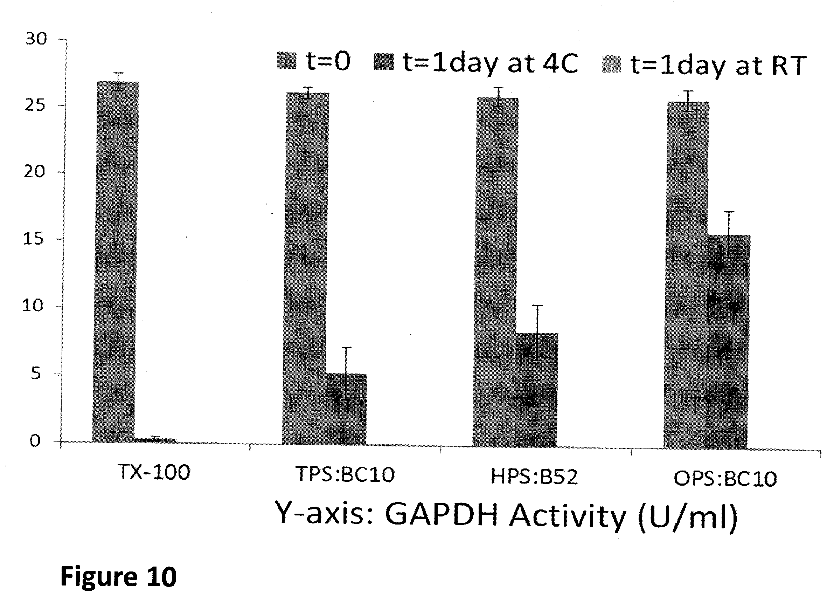

Skin tissue was collected from euthanized mice and stored at -80 °C until use. About 10 mg of each tissue was placed in a 2 ml tube. 400 µl of chilled lysis buffer (TX-1, TPS:Brij C10, HPS:Brij 52, and OPS:Brij C10) and protease inhibitor cocktail tablets (Roche Applied Science, IN, USA) were added to the tube (one tablet per 50 ml; one tablet contains Antipain-dihydrochloride 3mg, Aprotinin 0.5 mg, Bestatin 0.5 mg, Chymostatin 1mg, E-64 3 mg, EDTA-Na₂ 10 mg, Leupeptin 0.5 mg, Pefabloc SC 20 mg, Pepstatin 0.5 mg, and Phosphoramidon 3 mg). Tissue was homogenized using a homogenizer (IKA, NC, USA) at 13,000 rpm for 1 min. **Figure 9** illustrates the total protein recovered from homogenized mouse skin using TPS:Brij C10, HPS:Brij 52, and OPS:Brij C10, as compared to TX-1. **Figure 10** illustrates the specific GAPDH activity measured in the homogenized mouse skin.

To the extent that the term "includes" or "including" is used in the specification or the claims, it is intended to be inclusive in a manner similar to the term "comprising" as that term is interpreted when employed as a transitional word in a claim. As used in the specification and the claims, the singular forms "a," "an," and "the" include the plural. Furthermore, to the extent that the term "or" is employed (e.g., A or B), it is intended to mean "A or B or both." Finally, where the term "about" is used in conjunction with a number, it is intended to include ± 10% of the number. In other words, "about 10" may mean from 9 to 11.

As stated above, while the present application has been illustrated by the description of embodiments thereof, and while the embodiments have been described in considerable detail, it is not the intention of the applicants to restrict or in any way limit the scope of the appended claims to such detail. Additional advantages and modifications will readily appear to those skilled in the art, with the benefit of the disclosure provided in this application. Therefore, the application, in its broader aspects, is not limited to the specific details, illustrative examples shown, or any apparatus referred to.

## Claims

1. A composition, comprising:
at least one zwitterionic surfactant, represented by the following structural formula: 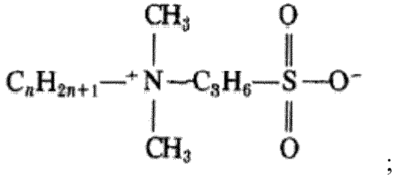 and
at least one non-ionic surfactant, represented by the following structural formula:
H(CH₂)ₐO(CH₂CH₂O)_{b}H,
wherein:
n is 14; and
a is 16 and b is 2 or 10.

2. The composition of claim 1, further comprising a buffer solution in which the at least one zwitterionic surfactant and the at least one non-ionic surfactant are dissolved.

3. The composition of claim 1, wherein the composition has a pH more basic than 7.0 in a buffer solution; optionally wherein the composition has a pH of between 7.0 and 9.0 in a buffer solution.

4. The composition of claim 1, further comprising a buffer solution that dissolves the zwitterionic surfactant and the non-ionic surfactant, the zwitterionic surfactant and the non-ionic surfactant comprising a total concentration of 0.01% to 10% w/v in the buffer solution; optionally the zwitterionic surfactant and the non-ionic surfactant comprising a total concentration of 0.01% to 5% w/v in the buffer solution; optionally the zwitterionic surfactant and the non-ionic surfactant comprising a total concentration of 0.1% to 2% w/v in the buffer solution; or optionally the zwitterionic surfactant and the non-ionic surfactant comprising a total concentration of 0.1% to 0.5% w/v in the buffer solution.

5. A non-surgical method for solubilizing, cosmetically remodeling and/or removing tissue on a patient's skin, the method comprising:
applying energy to a region of interest on the skin; and
contacting the region with a tissue solubilizing composition, the tissue solubilizing composition comprising:
at least one zwitterionic surfactant, represented by the following structural formula: 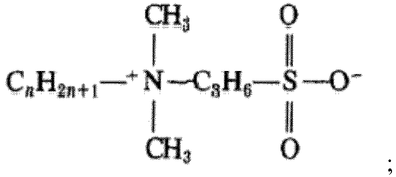 and
at least one non-ionic surfactant, represented by the following structural formula:
H(CH₂)ₐO(CH₂CH₂O)_{b}H,
wherein:
n is 14; and
a is 16 and b is 2 or 10.

6. The method of claim 5, further comprising:
removing at least a portion of the solubilized region of interest to leave an exposed area on the skin; and
applying a cosmetic composition to the exposed area.

7. The method of claim 6, the cosmetic composition comprising at least one of: elastin, an elastin-based peptide, collagen, a collagen-based peptide, resveratrol, idebenone, co-enzyme Q10, acetyl hexapeptide-3, glycosaminoglycans, palmitoyl pentapeptide-4, sodium hyaluronate, and combinations thereof.

8. The method of claim 5, the tissue solubilizing composition further comprising a buffer solution in which the at least one zwitterionic surfactant and the at least one non-ionic surfactant are dissolved.

9. The method of claim 5, the tissue solubilizing composition further comprising a buffer solution that dissolves the zwitterionic surfactant and the non-ionic surfactant, the zwitterionic surfactant and the non-ionic surfactant comprising a total concentration of 0.01% to 10% w/v in the buffer solution; optionally the zwitterionic surfactant and the non-ionic surfactant comprising a total concentration of 0.01% to 5% w/v in the buffer solution; optionally the zwitterionic surfactant and the non-ionic surfactant comprising a total concentration of 0.1% to 2% w/v in the buffer solution; or optionally the zwitterionic surfactant and the non-ionic surfactant comprising a total concentration of 0.1 % to 0.5% w/v in the buffer solution.

10. The method of claim 5, wherein the tissue solubilizing composition has a pH more basic than 7.0 in a buffer solution; optionally wherein the composition has a pH of between 7.0 and 9.0 in a buffer solution.

11. A tissue solubilizing composition for use in a surgical or diagnostic method practised on the human or animal body for recovering analytes from mucosal membrane, skin, or other tissue, the method comprising:
applying energy to a region of interest on the mucosal membrane, skin, or other tissue containing at least one analyte;
contacting the region with the tissue solubilizing composition, thereby solubilizing at least some of the mucosal membrane, skin, or other tissue containing at least one analyte; and
collecting the at least one analyte from the solubilized mucosal membrane, skin, and other tissue,
wherein the tissue solubilizing composition comprises:
at least one zwitterionic surfactant, represented by the following structural formula: 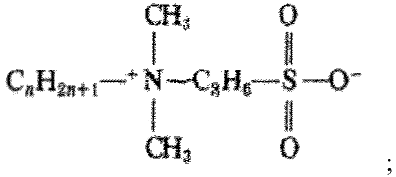 and
at least one non-ionic surfactant, represented by the following structural formula:
H(CH₂)ₐO(CH₂CH₂O)_{b}H,
wherein:
n is 14; and
a is 16 and b is 2 or 10.

12. The tissue solubilizing composition for use according to claim 11, wherein the analyte comprises one or more of: a protein, a cancer biomarker, an antibody, a peptide, a lipid, a nucleic acid, a small molecule, a microbe, a warfare agent, an environmental contaminant, or a drug.

13. The tissue solubilizing composition for use according to claim 11, the tissue solubilizing composition further comprising a buffer solution in which the at least one zwitterionic surfactant and the at least one non-ionic surfactant are dissolved.

14. The tissue solubilizing composition for use according to claim 11, the tissue solubilizing composition further comprising a buffer solution that dissolves the zwitterionic surfactant and the non-ionic surfactant, the zwitterionic surfactant and the non-ionic surfactant comprising a total concentration of 0.01% to 10% w/v in the buffer solution; optionally the zwitterionic surfactant and the non-ionic surfactant comprising a total concentration of 0.01% to 5% w/v in the buffer solution; optionally the zwitterionic surfactant and the non-ionic surfactant comprising a total concentration of 0.1% to 2% w/v in the buffer solution; or optionally the zwitterionic surfactant and the non-ionic surfactant comprising a total concentration of 0.1% to 0.5% w/v in the buffer solution.

15. The tissue solubilizing composition for use according to claim 11, wherein the tissue solubilizing composition has a pH more basic than 7.0 in a buffer solution; optionally wherein the composition has a pH of between 7.0 and 9.0 in a buffer solution.

16. A tissue solubilizing composition for use in a surgical or therapeutic method for solubilizing, remodeling and/or removing tissue on or beneath a patient's skin, the method comprising:
applying energy to a region of interest on the skin; and
contacting the region with the tissue solubilizing composition, the tissue solubilizing composition comprising:
at least one zwitterionic surfactant, represented by the following structural formula: 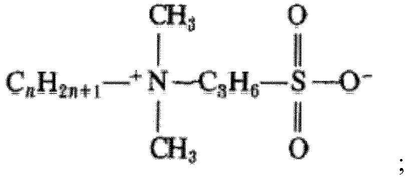 and
at least one non-ionic surfactant, represented by the following structural formula:
H(CH₂)ₐO(CH₂CH₂O)_{b}H,
wherein:
n is 14; and
a is 16 and b is 2 or 10.

17. The tissue solubilizing composition for use according to claim 16, the surgical or therapeutic method further comprising:
removing at least a portion of the solubilized region of interest to leave an exposed area on the skin; and
applying a therapeutic composition to the exposed area.

18. The tissue solubilizing composition for use iaccording to claim 17, the therapeutic composition comprising at least one of: a DNA-based drug, an RNA-based drug, a protein-based drug, a peptide-based drug, a lipid-based drug, a carbohydrate-based drug, a small molecule drug, a nanoparticle-based drug, and a liposome-encapsulated drug.

19. The tissue solubilizing composition according to claim 16, further comprising a buffer solution in which the at least one zwitterionic surfactant and at least one non-ionic surfactant are dissolved.

20. The tissue solubilizing composition for use according to claim 16, wherein the composition has a pH more basic than 7.0 in a buffer solution; optionally wherein the composition has a pH of between 7.0 and 9.0 in a buffer solution.

21. The solubilizing composition according to claim 16, further comprising a buffer solution that dissolves the zwitterionic surfactant and the non-ionic surfactant, the zwitterionic surfactant and the non-ionic surfactant comprising a total concentration of 0.01% to 10% w/v in the buffer solution; optionally the zwitterionic surfactant and the non-ionic surfactant comprising a total concentration of 0.01% to 5% w/v in the buffer solution; optionally the zwitterionic surfactant and the non-ionic surfactant comprising a total concentration of 0.1% to 2% w/v in the buffer solution; or optionally the zwitterionic surfactant and the non-ionic surfactant comprising a total concentration of 0.1% to 0.5% w/v in the buffer solution.

## Patentansprüche

1. Zusammensetzung, umfassend:
wenigstens ein zwitterionisches Tensid, dargestellt durch die folgende Strukturformel: 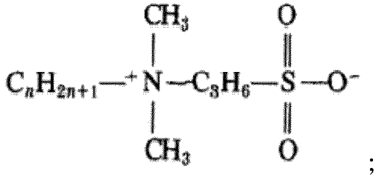 und
wenigstens ein nichtionisches Tensid, dargestellt durch die folgende Strukturformel:
H(CH₂)aO(CH₂CH₂O)_{b}H,
wobei:
n 14 ist; und
a 16 ist und b 2 oder 10 ist.

2. Zusammensetzung nach Anspruch 1, ferner umfassend eine Pufferlösung, in der das wenigstens eine zwitterionische Tensid und das wenigstens eine nichtionische Tensid gelöst sind.

3. Zusammensetzung nach Anspruch 1, wobei die Zusammensetzung in einer Pufferlösung einen basischen pH-Wert von über 7,0 aufweist; wobei optional die Zusammensetzung in einer Pufferlösung einen pH-Wert zwischen 7,0 und 9,0 aufweist.

4. Zusammensetzung nach Anspruch 1, ferner umfassend eine Pufferlösung, die das zwitterionische Tensid und das nichtionische Tensid löst, wobei das zwitterionische Tensid und das nichtionische Tensid eine Gesamtkonzentration von 0,01 bis 10 % Gew./Vol. in der Pufferlösung umfassen; wobei optional das zwitterionische Tensid und das nichtionische Tensid eine Gesamtkonzentration von 0,01 % bis 5 % Gew./Vol. in der Pufferlösung umfassen; oder wobei optional das zwitterionische Tensid und das nichtionische Tensid eine Gesamtkonzentration von 0,1 % bis 2 % Gew./Vol. in der Pufferlösung umfassen; oder wobei optional das zwitterionische Tensid und das nichtionische Tensid eine Gesamtkonzentration von 0,1 % bis 0,5 % Gew./Vol. in der Pufferlösung umfassen.

5. Nicht-chirurgisches Verfahren zum Solubilisieren, kosmetischen Umbilden und/oder Entfernen von Gewebe auf der Haut eines Patienten, wobei das Verfahren Folgendes umfasst:
Anwenden von Energie auf einen interessierenden Bereich auf der Haut; und
Kontaktieren des Bereichs mit einer gewebesolubilisierenden Zusammensetzung, wobei die gewebesolubilisierende Zusammensetzung Folgendes umfasst:
wenigstens ein zwitterionisches Tensid, dargestellt durch die folgende Strukturformel: 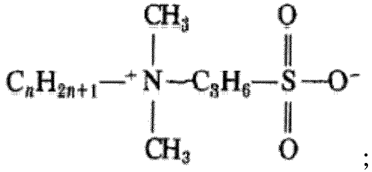 und
wenigstens ein nichtionisches Tensid, dargestellt durch die folgende Strukturformel:
H(CH₂)ₐO(CH₂CH₂O)_{b}H,
wobei:
n 14 ist; und
a 16 ist und b 2 oder 10 ist.

6. Verfahren nach Anspruch 5, ferner umfassend:
Entfernen von mindestens einem Teil des interessierenden solubilisierten Bereichs, um einen freiliegenden Bereich auf der Haut zu hinterlassen; und
Auftragen einer kosmetischen Zusammensetzung auf die freiliegende Fläche.

7. Verfahren nach Anspruch 6, wobei die kosmetische Zusammensetzung wenigstens eines von Elastin, ein auf Elastin basierendes Peptid, Kollagen, ein auf Kollagen basierendes Peptid, Resveratrol, Idebenon, Coenzym Q10, Acetylhexapeptid-3, Glycosaminoglycane, Palmitoylpentapeptid-4, Natriumhyaluronat und Kombinationen davon umfasst.

8. Verfahren nach Anspruch 5, wobei die gewebesolubilisierende Zusammensetzung ferner eine Pufferlösung umfasst, in der das wenigstens eine zwitterionische Tensid und das wenigstens eine nichtionische Tensid gelöst sind.

9. Verfahren nach Anspruch 5, wobei die gewebesolubilisierende Zusammensetzung ferner eine Pufferlösung umfasst, die das zwitterionische Tensid und das nichtionische Tensid löst, wobei das zwitterionische Tensid und das nichtionische Tensid eine Gesamtkonzentration von 0,01 % bis 10 % Gew./Vol. in der Pufferlösung umfassen; wobei optional das zwitterionische Tensid und das nichtionische Tensid eine Gesamtkonzentration von 0,01 % bis 5 % Gew./Vol. in der Pufferlösung umfassen; oder wobei optional das zwitterionische Tensid und das nichtionische Tensid eine Gesamtkonzentration von 0,1 % bis 2 % Gew./Vol. in der Pufferlösung umfassen; oder wobei optional das zwitterionische Tensid und das nichtionische Tensid eine Gesamtkonzentration von 0,1 % bis 0,5 % Gew./Vol. in der Pufferlösung umfassen.

10. Verfahren nach Anspruch 5, wobei die gewebesolubilisierende Zusammensetzung einen basischen pH-Wert von über 7,0 aufweist; wobei optional die Zusammensetzung in einer Pufferlösung einen pH-Wert zwischen 7,0 und 9,0 aufweist.

11. Gewebesolubilisierende Zusammensetzung zur Verwendung in einem chirurgischen oder diagnostischen Verfahren, das an einem menschlichen oder tierischen Körper zur Gewinnung von Analyten aus Schleimhaut, Haut oder anderem Gewebe durchgeführt wird, wobei das Verfahren Folgendes umfasst:
Anwendung von Energie auf einen interessierenden Bereich auf der Schleimhaut, Haut oder auf anderem Gewebe, das weningstens einen Analyten umfasst;
Kontaktieren des Bereichs mit der gewebesolubilisierenden Zusammensetzung, wodurch wenigstens einige der Schleimhaut, Haut oder des anderen Gewebes solubilisiert werden, die wenigstens einen Analyten enthalten; und
Erfassen des wenigstens einen Analyten aus der solubilisierten Schleimhaut, Haut und dem anderen Gewebe,
wobei die gewebesolubilisierende Zusammensetzung umfasst:
wenigstens ein zwitterionisches Tensid, dargestellt durch die folgende Strukturformel: 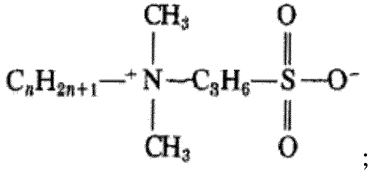 und
wenigstens ein nichtionisches Tensid, dargestellt durch die folgende Strukturformel:
H(CH₂)ₐO(CH₂CH₂O)_{b}H,
wobei:
n 14 ist; und
a 16 ist und b 2 oder 10 ist.

12. Gewebesolubilisierende Zusammensetzung zur Verwendung nach Anspruch 11, wobei der Analyt eines oder mehr von Protein, Krebsbiomarker, Antikörper, Peptid, Lipid, Nukleinsäure, kleines Molekül, Mikrobe, Kampfmittel, Umweltschadstoff oder Medikament umfasst.

13. Gewebesolubilisierende Zusammensetzung zur Verwendung nach Anspruch 11, wobei die gewebesolubilisierende Zusammensetzung ferner eine Pufferlösung umfasst, in der das wenigstens eine zwitterionische Tensid und das wenigstens eine nichtionische Tensid gelöst sind.

14. Gewebesolubilisierende Zusammensetzung zur Verwendung nach Anspruch 11, wobei die gewebesolubilisierende Zusammensetzung ferner eine Pufferlösung umfasst, die das zwitterionische Tensid und das nichtionische Tensid löst, wobei das zwitterionische Tensid und das nichtionische Tensid eine Gesamtkonzentration von 0,01 % bis 10 % Gew./Vol. in der Pufferlösung umfassen; wobei optional das zwitterionische Tensid und das nichtionische Tensid eine Gesamtkonzentration von 0,01 % bis 5 % Gew./Vol. in der Pufferlösung umfassen; oder wobei optional das zwitterionische Tensid und das nichtionische Tensid eine Gesamtkonzentration von 0,1 % bis 2 % Gew./Vol. in der Pufferlösung umfassen; oder wobei optional das zwitterionische Tensid und das nichtionische Tensid eine Gesamtkonzentration von 0,1 % bis 0,5 % Gew./Vol. in der Pufferlösung umfassen.

15. Gewebesolubilisierende Zusammensetzung zur Verwendung nach Anspruch 11, wobei die gewebesolubilisierende Zusammensetzung einen basischen pH-Wert von über 7,0 aufweist; wobei optional die Zusammensetzung in einer Pufferlösung einen pH-Wert zwischen 7,0 und 9,0 aufweist.

16. Gewebesolubilisierende Zusammensetzung zur Verwendung in einem chirurgischen oder therapeutischen Verfahren zum Solubilisieren, Umbilden und/oder Entfernen von Gewebe auf oder unter der Haut eines Patienten, wobei das Verfahren Folgendes umfasst:
Anwenden von Energie auf einen interessierenden Bereich auf der Haut; und Kontaktieren des Bereichs mit der gewebesolubilisierenden Zusammensetzung, wobei die gewebesolubilisierende Zusammensetzung Folgendes umfasst:
wenigstens ein zwitterionisches Tensid, dargestellt durch die folgende Strukturformel: 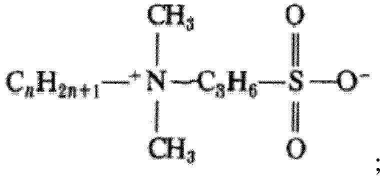 und
wenigstens ein nichtionisches Tensid, dargestellt durch die folgende Strukturformel:
H(CH₂)ₐO(CH₂CH₂O)_{b}H,
wobei:
n 14 ist; und
a 16 ist und b 2 oder 10 ist.

17. Gewebesolubilisierende Zusammensetzung zur Verwendung nach Anspruch 16, wobei das chirurgische oder therapeutische Verfahren ferner Folgendes umfasst:
Entfernen von wenigstens einem Teil des interessierenden solubilisierten Bereichs, um einen freiliegenden Bereich auf der Haut zu hinterlassen; und
Auftragen einer therapeutischen Zusammensetzung auf die freiliegende Fläche.

18. Gewebesolubilisierende Zusammensetzung zur Verwendung nach Anspruch 17, wobei die therapeutische Zusammensetzung wenigstens eines von Arzneimittel auf DNA-Basis, Arzneimittel auf RNA-Basis, Arzneimittel auf Proteinbasis, Arzneimittel auf Peptidbasis, Arzneimittel auf Lipidbasis, auf Kohlenhydraten basierendes Medikament, niedermolekularem Medikament, Medikament auf Nanopartikelbasis und mit Liposomen verkapseltem Medikament umfasst.

19. Gewebesolubilisierende Zusammensetzung nach Anspruch 16, ferner umfassend eine Pufferlösung, in der das wenigstens eine zwitterionische Tensid und das wenigstens eine nichtionische Tensid gelöst sind.

20. Gewebesolubilisierende Zusammensetzung zur Verwendung nach Anspruch 16, wobei die Zusammensetzung einen basischen pH-Wert von mehr als 7,0 in einer Pufferlösung aufweist; wobei optional die Zusammensetzung in einer Pufferlösung einen pH-Wert zwischen 7,0 und 9,0 aufweist.

21. Solubilisierende Zusammensetzung nach Anspruch 16, ferner umfassend eine Pufferlösung, in der das zwitterionische Tensid und das nichtionische Tensid gelöst werden, wobei das zwitterionische Tensid und das nichtionische Tensid eine Gesamtkonzentration von 0,01 bis 10 % Gew./Vol. in der Pufferlösung umfassen; wobei optional das zwitterionische Tensid und das nichtionische Tensid eine Gesamtkonzentration von 0,01 % bis 5 % Gew./Vol. in der Pufferlösung umfassen; oder wobei optional das zwitterionische Tensid und das nichtionische Tensid eine Gesamtkonzentration von 0,1 % bis 2 % Gew./Vol. in der Pufferlösung umfassen; oder wobei optional das zwitterionische Tensid und das nichtionische Tensid eine Gesamtkonzentration von 0,1 % bis 0,5 % Gew./Vol. in der Pufferlösung umfassen.

## Revendications

1. Composition, comprenant :
au moins un tensioactif zwittérionique, représenté par la formule structurale suivante : 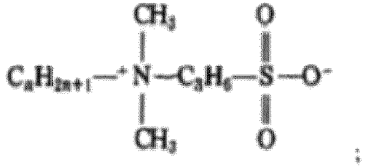 et
au moins un tensioactif non ionique, représenté par la formule structurale suivante :
H(CH₂)ₐO(CH₂CH₂O)_{b}H,
dans lesquelles :
n vaut 14; et
a vaut 16 et b vaut 2 ou 10.

2. Composition selon la revendication 1, comprenant en outre une solution tampon dans laquelle le au moins un tensioactif zwittérionique et le au moins un tensioactif non ionique sont dissous.

3. Composition selon la revendication 1, dans laquelle la composition a un pH plus basique que 7,0 dans une solution tampon ; éventuellement où la composition a un pH compris entre 7,0 et 9,0 dans une solution tampon.

4. Composition selon la revendication 1, qui comprend en outre une solution tampon qui dissout le tensioactif zwittérionique et le tensioactif non ionique, le tensioactif zwittérionique et le tensioactif non ionique comprenant une concentration totale de 0,01 % à 10 % p/v dans la solution tampon ; éventuellement le tensioactif zwittérionique et le tensioactif non ionique comprenant une concentration totale de 0,01 % à 5 % p/v dans la solution tampon ; éventuellement le tensioactif zwittérionique et le tensioactif non ionique comprenant une concentration totale de 0,1 % à 2 % p/v dans la solution tampon ; ou éventuellement le tensioactif zwittérionique et le tensioactif non ionique comprenant une concentration totale de 0,1 % à 0,5 % p/v dans la solution tampon.

5. Procédé non chirurgical pour solubiliser, remodeler cosmétiquement et/ou enlever un tissu sur la peau d'un patient, le procédé comprenant :
l'application d'énergie à une région d'intérêt sur la peau ; et
la mise en contact de la région avec une composition de solubilisation de tissu, la composition de solubilisation de tissu comprenant :
au moins un tensioactif zwittérionique représenté par la formule structurale suivante : 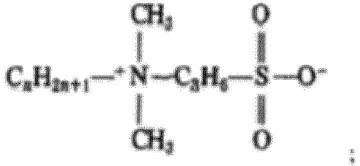 et
au moins un tensioactif non ionique, représenté par la formule structurale suivante :
H(CH₂)ₐO(CH₂CH₂O)_{b}H,
dans lesquelles :
n vaut 14; et
a vaut 16 et b vaut 2 ou 10.

6. Procédé selon la revendication 5, comprenant en outre :
l'enlèvement d'au moins une partie de la région d'intérêt solubilisée pour laisser une surface exposée sur la peau ; et
l'application d'une composition cosmétique à la surface exposée.

7. Procédé selon la revendication 6,
la composition cosmétique comprenant au moins un de : l'élastine, un peptide à base d'élastine, le collagène, un peptide à base de collagène, le resvératrol, l'idébénone, le co-enzyme Q10, l'acétyl hexapeptide-3, des glycosaminoglycanes, le palmitoyl pentapeptide-4, le hyaluronate de sodium et leurs combinaisons.

8. Procédé selon la revendication 5, la composition de solubilisation de tissu comprenant en outre une solution tampon dans laquelle le au moins un tensioactif zwittérionique et le au moins un tensioactif non ionique sont dissous.

9. Procédé selon la revendication 5, la composition de solubilisation de tissu comprenant en outre une solution tampon qui dissout le tensioactif zwittérionique et le tensioactif non ionique, le tensioactif zwittérionique et le tensioactif non ionique comprenant une concentration totale de 0,01 % à 10 % p/v dans la solution tampon ; éventuellement le tensioactif zwittérionique et le tensioactif non ionique comprenant une concentration totale de 0,01 % à 5 % p/v dans la solution tampon ; éventuellement le tensioactif zwittérionique et le tensioactif non ionique comprenant une concentration totale de 0,1 % à 2 % p/v dans la solution tampon ; ou éventuellement le tensioactif zwittérionique et le tensioactif non ionique comprenant une concentration totale de 0,1 % à 0,5 % p/v dans la solution tampon.

10. Procédé selon la revendication 5, dans lequel la composition de solubilisation de tissu a un pH plus basique que 7,0 dans une solution tampon ; éventuellement où la composition a un pH compris entre 7,0 et 9,0 dans une solution tampon.

11. Composition de solubilisation de tissu destinée à être utilisée dans un procédé chirurgical ou de diagnostic pratiqué sur le corps humain ou animal pour récupérer des analytes de la membrane muqueuse, de la peau ou d'un autre tissu, le procédé comprenant :
l'application d'énergie à une région d'intérêt sur la membrane muqueuse, la peau ou un autre tissu contenant au moins un analyte ;
la mise en contact de la région avec la composition de solubilisation de tissu, solubilisant ainsi au moins une partie de la membrane muqueuse, de la peau ou d'un autre tissu contenant au moins un analyte ; et
la collecte de l'au moins un analyte de la membrane muqueuse, de la peau et d'un autre tissus solubilisé,
où la composition de solubilisation de tissu comprend :
au moins un tensioactif zwittérionique, représenté par la formule structurale suivante : 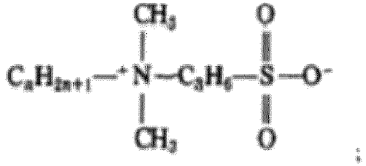 et
au moins un tensioactif non ionique, représenté par la formule structurale suivante :
H(CH₂)ₐO(CH₂CH₂O)_{b}H,
dans lesquelles :
n vaut 14; et
a vaut 16 et b vaut 2 ou 10.

12. Composition de solubilisation de tissu destinée à être utilisée selon la revendication 11, dans laquelle l'analyte comprend un ou plusieurs de : une protéine, un biomarqueur de cancer, un anticorps, un peptide, un lipide, un acide nucléique, une petite molécule, un microbe, un agent de combat, un contaminant environnemental ou un médicament.

13. Composition de solubilisation de tissu destinée à être utilisée selon la revendication 11, comprenant en outre une solution tampon dans laquelle le au moins un tensioactif zwittérionique et le au moins un tensioactif non ionique sont dissous.

14. Composition de solubilisation de tissu destinée à être utilisée selon la revendication 11, la composition de solubilisation de tissu comprenant en outre une solution tampon qui dissout le tensioactif zwittérionique et le tensioactif non ionique, le tensioactif zwittérionique et le tensioactif non ionique comprenant une concentration totale de 0,01 % à 10 % p/v dans la solution tampon ; éventuellement le tensioactif zwittérionique et le tensioactif non ionique comprenant une concentration totale de 0,01 % à 5 % p/v dans la solution tampon ; éventuellement le tensioactif zwittérionique et le tensioactif non ionique comprenant une concentration totale de 0,1 % à 2 % p/v dans la solution tampon ; ou éventuellement le tensioactif zwittérionique et le tensioactif non ionique comprenant une concentration totale de 0,1 % à 0,5 % p/v dans la solution tampon.

15. Composition de solubilisation de tissu destinée à être utilisée selon la revendication 11, dans laquelle la composition de solubilisation de tissu a un pH plus basique que 7,0 dans une solution tampon ; éventuellement où la composition a un pH compris entre 7,0 et 9,0 dans une solution tampon.

16. Composition de solubilisation de tissu destinée à être utilisée dans un procédé chirurgical ou thérapeutique pour solubiliser, remodeler et/ou enlever un tissu sur ou sous la peau d'un patient, le procédé comprenant :
l'application d'énergie à une région d'intérêt sur la peau ; et
la mise en contact de la région avec la composition de solubilisation de tissu, la composition de solubilisation de tissu comprenant :
au moins un tensioactif zwittérionique, représenté par la formule structurale suivante : 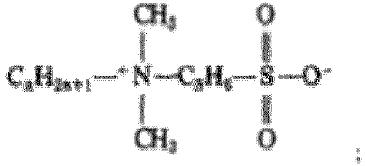 et
au moins un tensioactif non ionique, représenté par la formule structurale suivante :
H(CH₂)ₐO(CH₂CH₂O)_{b}H,
dans lesquelles :
n vaut 14; et
a vaut 16 et b vaut 2 ou 10.

17. Composition de solubilisation de tissu destinée à être utilisée selon la revendication 16, le procédé chirurgical ou thérapeutique comprenant en outre :
l'enlèvement d'au moins une partie de la région d'intérêt solubilisée pour laisser une surface exposée sur la peau ; et
l'application d'une composition thérapeutique à la surface exposée.

18. Composition de solubilisation de tissu destinée à être utilisée selon la revendication 17, la composition thérapeutique comprenant au moins un de : un médicament à base d'ADN, un médicament à base d'ARN, un médicament à base de protéine, un médicament à base de peptide, un médicament à base de lipide, un médicament à base de glucide, un médicament petite molécule, un médicament à base de nanoparticule et un médicament encapsulé dans un liposome.

19. Composition de solubilisation de tissu selon la revendication 16, comprenant en outre une solution tampon dans laquelle le au moins un tensioactif zwittérionique et le au moins un tensioactif non ionique sont dissous.

20. Composition de solubilisation de tissu destinée à être utilisée selon la revendication 16, dans laquelle la composition a un pH plus basique que 7,0 dans une solution tampon ; éventuellement où la composition a un pH compris entre 7,0 et 9,0 dans une solution tampon.

21. Composition de solubilisation de tissu selon la revendication 16, comprenant en outre une solution tampon qui dissout le tensioactif zwittérionique et le tensioactif non ionique, le tensioactif zwittérionique et le tensioactif non ionique comprenant une concentration totale de 0,01 % à 10 % p/v dans la solution tampon ; éventuellement le tensioactif zwittérionique et le tensioactif non ionique comprenant une concentration totale de 0,01 % à 5 % p/v dans la solution tampon ; éventuellement le tensioactif zwittérionique et le tensioactif non ionique comprenant une concentration totale de 0,1 % à 2 % p/v dans la solution tampon ; ou éventuellement le tensioactif zwittérionique et le tensioactif non ionique comprenant une concentration totale de 0,1 % à 0,5 % p/v dans la solution tampon.
